(19) Europäisches Patentamt
European Patent Office
Office européen des brevets

(11) **EP 4 744 598 A1**

(12) **EUROPEAN PATENT APPLICATION**
published in accordance with Art. 153(4) EPC

(43) Date of publication:
**20.05.2026 Bulletin 2026/21**

(21) Application number: **24839547.7**

(22) Date of filing: **28.06.2024**

(51) International Patent Classification (IPC):
***A61B 8/00*** (2006.01)     ***A61N 7/02*** (2006.01)

(52) Cooperative Patent Classification (CPC):
**A61B 8/00; A61N 7/02**

(86) International application number:
**PCT/JP2024/023518**

(87) International publication number:
**WO 2025/013655 (16.01.2025 Gazette 2025/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.07.2023 JP 2023113658**

(71) Applicant: **SONIRE THERAPEUTICS INC.
Tokyo 103-0023 (JP)**

(72) Inventor: **NEMOTO, Kazuhito
Tokyo 103-0023 (JP)**

(74) Representative: **Novagraaf International SA
Chemin de l'Echo 3
1213 Onex, Geneva (CH)**

(54) **HIFU IRRADIATION DEVICE AND METHOD FOR EVALUATING STATE OF SAME**

(57)     The purpose of the present invention is to evaluate the effect of ultrasound transmitted by a therapeutic ultrasonic vibrator on an ultrasonic imaging probe. A control unit (22) executes an ultrasound transmission process, an ultrasound reception process, and a distribution measurement process. The ultrasound transmission process is a process for causing a therapeutic ultrasonic vibrator (14) to transmit evaluation ultrasound having a lower intensity than therapeutic ultrasound. The ultrasound reception process is a process for acquiring, from each vibrator (28), a reception signal based on the ultrasound received by each vibrator (28). The distribution measurement process is a process for measuring, on the basis of the reception signals acquired from the vibrators (28), the intensity distribution of the ultrasound arriving at the plurality of vibrators (28).

FIG. 1

**Description**

TECHNICAL FIELD

The present disclosure relates to a HIFU sonication apparatus having an ultrasound imaging probe, and in particular to evaluation of a state of such a HIFU sonication apparatus.

BACKGROUND

Therapeutic apparatuses which employ high intensity focused ultrasound (HIFU) are widely in use. Such therapeutic apparatuses, referred to as a HIFU sonication apparatus or a HIFU sonication system, sonicate(insonate) a focused ultrasound to a therapeutic site to necrotize a biological tissue.

In general, a HIFU sonication apparatus has a plurality of ultrasound transducers for therapy, which are disposed on a concave surface. The plurality of ultrasound transducers are disposed in such an arrangement that ultrasounds emitted from the ultrasound transducers are sonicated toward one point, and form a focal point. During therapy, the ultrasounds are sonicated such that the position of the focal point coincides with the therapeutic site. For checking the position of sonication, an ultrasound diagnostic apparatus which shows the focal point on an ultrasound image is utilized.

Patent Literature 1 discloses an ultrasound therapeutic apparatus which observes a position of the focal point using an ultrasound diagnostic apparatus which displays a B mode image (tomographic image). In this apparatus, an ultrasound of sufficiently low power to not affect the tissues is emitted from the ultrasound transducer for therapy, and a tomographic image is displayed through transmission and reception of the ultrasound by an ultrasound imaging probe. Because acoustic characteristics of a tissue of a patient change with changes in temperature of the tissue, the position of the focal point is shown in the tomographic image by a magnitude of brightness.

CITATION LIST

PATENT LITERATURE

  [Patent Literature 1] JP H8-71069 A
  [Patent Literature 2] JP 2014-30509 A

SUMMARY

TECHNICAL PROBLEM

With a HIFU sonication apparatus, there may be cases in which the ultrasound for therapy is reflected in the body of the patient or by a surface of the body of the patient, the reflected waves are concentrated at a region near the ultrasound imaging probe, and thermal stress is thus applied to the ultrasound imaging probe. In consideration of this, Patent Literature 2 describes a technique in which, in an ultrasound diagnostic apparatus having a probe for therapy and a probe for imaging (ultrasound imaging probe), the influences experienced by an ultrasound receiving surface of the diagnostic probe by the reflected wave of the therapeutic ultrasound sonicated from the probe for therapy are computed.

In the related art such as that described in Patent Literature 2, when the influence on the ultrasound imaging probe received by the ultrasound for therapy is evaluated, an ultrasound having an intensity similar to that during the therapy is transmitted from the ultrasound transducer for therapy. With this technique, thermal stress from the ultrasound is also applied to the ultrasound imaging probe during the evaluation, such that there may be cases where the lifetime of the ultrasound imaging probe is shortened.

An advantage of the present disclosure lies in enabling evaluation of the influence on the ultrasound imaging probe received from the ultrasound transmitted by the ultrasound transducer for therapy.

SOLUTION TO PROBLEM

According to one aspect of the present disclosure, there is provided a HIFU sonication apparatus comprising a therapeutic ultrasound transducer that transmits a therapeutic ultrasound; an ultrasound probe having a plurality of transducers; and a controller configured to control the therapeutic ultrasound transducer and the ultrasound probe, wherein the controller is configured to execute an ultrasound transmission process to cause the therapeutic ultrasound transducer to transmit an evaluation ultrasound having lower intensity than that of the therapeutic ultrasound; an ultrasound reception process to acquire from each of the transducers a reception signal based on an ultrasound received by each of the transducers; and a distribution measurement process to measure an intensity distribution of ultrasounds arriving at the plurality of transducers, based on the reception signal acquired from each of the transducers, and the controller is configured to execute a process to determine a degree of concentration of the ultrasound received by each of the transducers, based on the intensity distribution in regard to a direction of arrangement of the plurality of transducers.

Desirably, the intensity distribution is represented by a distribution function having one peak for a value on a vertical axis with respect to a horizontal axis which extends in the direction of arrangement of the plurality of transducers, and the controller is configured to execute an evaluation process to evaluate an influence of the therapeutic ultrasound on each of the transducers by approximation parameters which define the distribution function.

According to another aspect of the present disclosure, there is provided a HIFU sonication apparatus comprising a therapeutic ultrasound transducer that transmits a therapeutic ultrasound; an ultrasound probe having a plurality of transducers; and a controller configured to control the therapeutic ultrasound transducer and the ultrasound probe, wherein the controller is configured to execute an ultrasound transmission process to cause the therapeutic ultrasound transducer to transmit an evaluation ultrasound having lower intensity than that of the therapeutic ultrasound; an ultrasound reception process to acquire from each of the transducers a reception signal based on an ultrasound received by each of the transducers; and a distribution measurement process to measure an intensity distribution of ultrasounds arriving at the plurality of transducers, based on the reception signal acquired from each of the transducers, the intensity distribution is represented by a distribution function having one peak for a value on a vertical axis with respect to a horizontal axis which extends in a direction of arrangement of the plurality of transducers, and the controller is configured to execute an evaluation process to evaluate an influence of the therapeutic ultrasound on each of the transducers by approximation parameters which define the distribution function.

Desirably, the distribution function includes a Gaussian function or a Lorentz function.

According to another aspect of the present disclosure, there is provided a method of evaluating a state of an HIFU sonication apparatus comprising a therapeutic ultrasound transducer which transmits a therapeutic ultrasound, and an ultrasound probe having a plurality of transducers, the method comprising causing the therapeutic ultrasound transducer to transmit an evaluation ultrasound having lower intensity than that of the therapeutic ultrasound; acquiring from each of the transducers a reception signal based on an ultrasound received by each of the transducers; measuring an intensity distribution of ultrasounds arriving at the plurality of transducers, based on the reception signal acquired from each of the transducers; and determining a degree of concentration of the ultrasound received by each of the transducers, based on the intensity distribution in regard to a direction of arrangement of the plurality of transducers.

Desirably, the intensity distribution is represented by a distribution function having one peak for a value on a vertical axis with respect to a horizontal axis which extends in the direction of arrangement of the plurality of transducers, and the method further comprises evaluating an influence of the therapeutic ultrasound on each of the transducers by approximation parameters which define the distribution function. According to another aspect of the present disclosure, there is provided a method of evaluating a state of a HIFU sonication apparatus comprising a therapeutic ultrasound transducer which transmits a therapeutic ultrasound, and an ultrasound probe having a plurality of transducers, the method comprising causing the therapeutic ultrasound transducer to transmit an evaluation ultrasound having lower intensity than that of the therapeutic ultrasound; acquiring from each of the transducers a reception signal based on an ultrasound received by each of the transducers; and measuring an intensity distribution of ultrasounds arriving at the plurality of transducers, based on the reception signal acquired from each of the transducers, wherein the intensity distribution is represented by a distribution function having one peak for a value on a vertical axis with respect to a horizontal axis which extends in a direction of arrangement of the plurality of transducers, and the method further comprises evaluating an influence of the therapeutic ultrasound on each of the transducers by approximation parameters which define the distribution function. Desirably, the distribution function includes a Gaussian function or a Lorentz function.

ADVANTAGEOUS EFFECTS OF INVENTION

According to an aspect of the present disclosure, the influence, received by the ultrasound imaging probe, of the ultrasound transmitted from the ultrasound transducer for therapy can be evaluated.

BRIEF DESCRIPTION OF DRAWINGS

FIG. 1 is a diagram showing a structure of a HIFU sonication apparatus.
FIG. 2 is a diagram schematically showing n transducers provided in an ultrasound imaging probe.
FIG. 3 is a diagram for explaining an example of a phenomenon which is caused at a region near the ultrasound imaging probe.

FIG. 4 is a diagram showing an example of fitting of a distribution function I(x) to a plurality of measurement values measured for a plurality of transducers.

FIG. 5 is a diagram showing another example of fitting of a distribution function I(x) to a plurality of measurement values measured for a plurality of transducers.

DESCRIPTION OF EMBODIMENTS

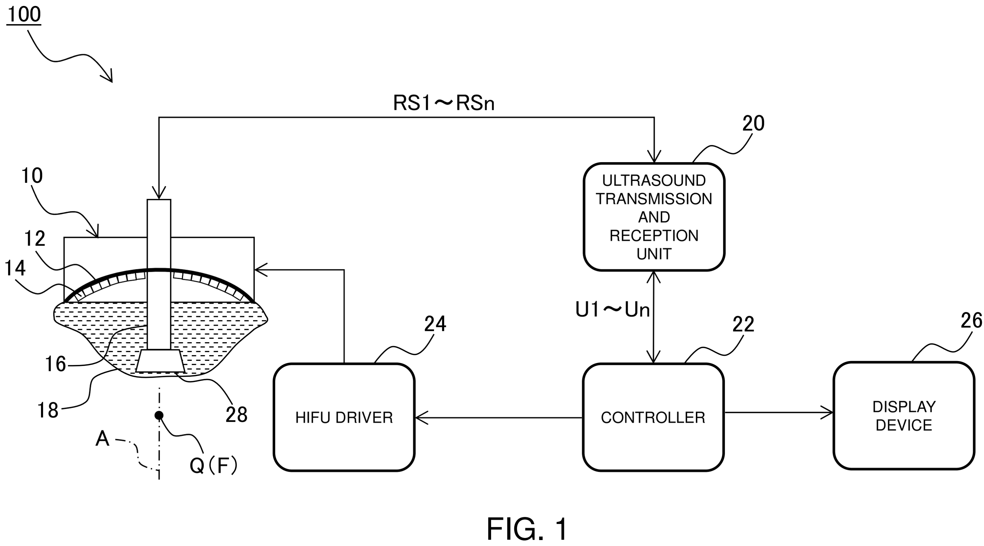

FIG. 1 shows a structure of a HIFU sonication apparatus 100 according to an embodiment of the present disclosure. The HIFU sonication apparatus 100 comprises a HIFU transducer unit 10, an ultrasound imaging probe 16, a coupling bag 18, an ultrasound transmission and reception unit 20, a controller 22, a HIFU driver 24, and a display device 26. In the following description, for simplification, the "ultrasound imaging probe 16" will be simply referred to as an "ultrasound probe 16".

The HIFU transducer unit 10 comprises a housing 12 of a container shape with an opening directed downward, and a plurality of therapeutic ultrasound transducers 14 fixed on the housing 12. The plurality of therapeutic ultrasound transducers 14 are disposed, for example, on a virtual curved surface which is convex upward, and are fixed on the housing 12 in such a manner that, when the therapeutic ultrasound transducers 14 emit ultrasounds, the intensity of the ultrasounds is increased at a therapeutic reference point Q below the housing 12. That is, the therapeutic ultrasound transducers 14 are fixed on the housing 12 so as to enable forming a focal point F on the therapeutic reference point Q.

The ultrasound probe 16 is attached to the housing 12 in such a manner that the ultrasound is transmitted and received at a position below the housing 12 and above the therapeutic reference point Q. In the present embodiment, the ultrasound probe 16 penetrates through a vertex part of the housing 12 in an up-and-down direction, and a tip portion thereof which transmits and receives the ultrasound is directed downward. A plurality of imaging ultrasound transducers 28 are disposed at the tip portion of the ultrasound probe 16. The ultrasound probe 16 may be movable in an up-and-down direction. In addition, the ultrasound probe 16 may be rotatable about an axis in a longitudinal direction. In the following description, for simplification, the "imaging ultrasound transducer 28" will be simply referred to as a "transducer 28".

Below the HIFU transducer unit 10, the coupling bag 18, for matching acoustic impedance between the therapeutic ultrasound transducers 14 and the patient and between the ultrasound probe 16 and the patient, is provided. The coupling bag 18 may be a bag filled with a liquid such as water.

The controller 22 may be formed from a computing device such as a personal computer, a tablet computer, or the like. Alternatively, one or a plurality of computing devices may form the controller 22. A manipulation device (not shown) for allowing a user to manipulate the HIFU sonication apparatus 100 is connected to a plurality of controllers 22. The manipulation device may include a mouse, a touch panel integrated with the display device 26, a switch, a keyboard, or the like.

The HIFU driver 24 may include an electronic circuit which drives the therapeutic ultrasound transducers 14 to generate ultrasounds. The HIFU driver 24 causes each of the therapeutic ultrasound transducers 14 to generate an ultrasound according to control by the controller 22. In addition, the HIFU driver 24 adjusts intensity of the ultrasound generated by each of the therapeutic ultrasound transducers 14 according to control by the controller 22.

The ultrasound transmission and reception unit 20 includes an electronic circuit which outputs an electric signal to the plurality of transducers 28 of the ultrasound probe 16, and acquires an electric signal which is output from the transducer 28. The ultrasound transmission and reception unit 20 executes the following processes according to control by the controller 22. Specifically, the ultrasound transmission and reception unit 20 causes the ultrasound probe 16 to transmit an imaging ultrasound, and scans the beam by the imaging ultrasound which is transmitted (ultrasound beam). The ultrasound beam is scanned on an observation plane including a center axis A extending from an uppermost part of the housing 12 in the up-and-down direction. The ultrasound transmission and reception unit 20 causes the ultrasound probe 16 to receive a reflected ultrasound arriving from a direction to which the ultrasound beam is directed, acquires a reception signal based on the reflected ultrasound received from each direction to which the ultrasound beam is directed, and outputs the reception signal to the controller 22.

The controller 22 produces ultrasound data based on the reception signal acquired on the observation plane. The ultrasound data may be sonication region data indicating a region at which a harmonic component is generated in a biological tissue of the patient (cavitation region), or B mode image data indicating a B mode image (tomographic image) acquired for the biological tissue of the patient.

Before the therapeutic ultrasound is sonicated from the HIFU transducer unit 10 to the patient, for example, the following positioning process is executed. The HIFU driver 24 causes each of the therapeutic ultrasound transducers 14 to transmit an ultrasound having a lower intensity than that during the therapy. Controlled by the controller 22, the ultrasound transmission and reception unit 20 causes the ultrasound probe 16 to scan the ultrasound beam on the observation plane, acquires the B mode image data as ultrasound data, and outputs the B mode image data to the controller 22. The controller 22 causes the display device 26 to display a B mode image. The user, in this example a surgeon, refers to the B mode image displayed on the display device 26, and checks for any variation of the focal point F of the ultrasound transmitted from the HIFU transducer unit 10 from the position of a diseased tissue.

When a difference between the position of the focal point F and that of the diseased tissue exceeds a tolerable range, the user changes positions or orientations of the ultrasound probe 16 and the HIFU transducer unit 10. After the user confirms that the position of the focal point F coincides with the position of the diseased tissue, the user performs a manipulation for therapy using the controller 22. The controller 22 controls the HIFU driver 24 in response to the manipulation by the user. The HIFU driver 24 causes the therapeutic ultrasound transducers 14 to transmit the therapeutic ultrasounds having intensities necessary for therapy, according to the control by the controller 22. In this manner, the biological tissue is cauterized at the focal point F, and the therapy is applied.

The HIFU sonication apparatus 100 executes a process to display an image indicating the sonication region to which the therapy ultrasound is sonicated, in an overlapping manner with the B mode image while sonicating the therapeutic ultrasound to the patient. The process to overlappingly display two images may be a process to produce new image data by combining image data by making one image transparent so that the other image can be viewed, and to display an image based on the new image data.

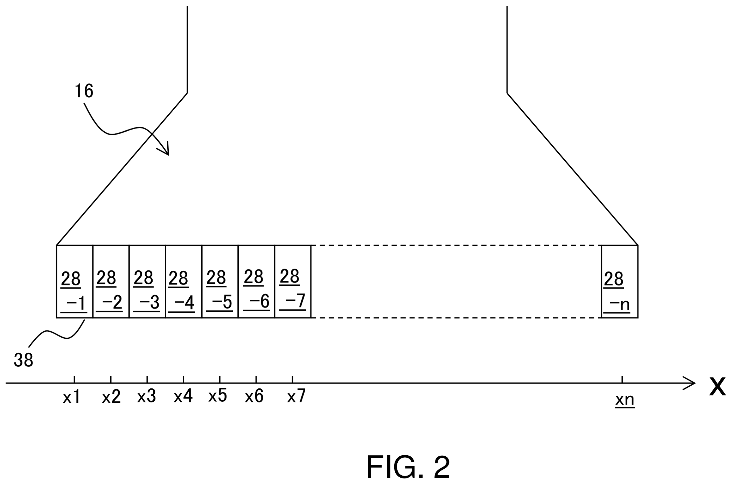

FIG. 2 schematically shows n transducers 28-1 to 28-n provided on the ultrasound probe 16. The transducers 28-1 to 28-n are arranged in a straight line on a transmission and reception surface 38 of the ultrasound probe 16 along a long axis direction (x axis direction) in the order of transducers 28-1, 28-2, 28-3, ... 28-n. The positions of the transducers 28-1, 28-2, 28-3, ... 28-n on the x axis are respectively x = x1, x2, x3, ... xn. Each of the transducers 28-1 to 28-n transmits an ultrasound from the transmission and reception surface 38, and receives an ultrasound arriving at the transmission and reception surface 38.

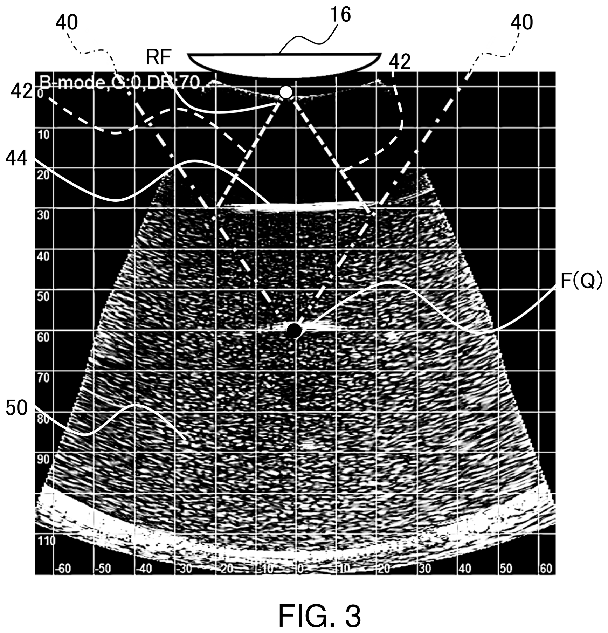

FIG. 3 is a diagram for conceptually explaining an example of a phenomenon which appears in a region near the ultrasound probe 16, on a B mode image 50. A reflecting tissue 44 which reflects the ultrasound is present between the ultrasound probe 16 and the focal point F of the therapeutic ultrasound (therapeutic reference point Q). In the example configuration illustrated in FIG. 3, the reflecting tissue 44 forms a reflecting surface which extends in a lateral direction. The therapeutic ultrasound primarily propagates in a region surrounded by two virtual straight lines 40 which intersect at the focal point F. The ultrasound reflected by the reflecting tissue 44 (reflected wave) propagates upward, and toward a reflected wave focal point RF near the transmission and reception surface 38 of the ultrasound probe 16. The reflected wave primarily propagates in a region surrounded by two virtual straight lines 42. The straight line 42 at the left extends from the intersection between the reflecting surface of the reflecting tissue 44 and the left straight line 40 toward upper right, at the same angle as an angle between the straight line 40 and the reflecting surface. The straight line 42 at the right extends from the intersection between the reflecting surface of the reflecting tissue 44 and the right straight line 40 toward upper left, at the same angle as an angle between the straight line 40 and the reflecting surface. The left and right straight lines 42 intersect at the reflected wave focal point RF at a region extending from the reflecting surface.

In this manner, when the reflecting tissue 44 is present between the ultrasound probe 16 and the focal point F of the therapeutic ultrasound, there may be cases in which the therapeutic ultrasound is reflected by the reflecting tissue 44 upward, toward the reflected wave focal point RF, and energy of the reflected wave is concentrated at the reflected wave focal point RF. Of the plurality of transducers 28 of the ultrasound probe 16, transducers 28 near the reflected wave focal point RF experience thermal stress by the reflected wave, possibly resulting in shortened lifetime or inability to perform to their inherent capability.

In consideration of this, in the HIFU sonication apparatus 100 according to the present embodiment, an evaluation process described below may be executed after the positioning process is completed and before the therapeutic ultrasound is sonicated from the HIFU transducer unit 10 to the patient. In the evaluation process, transducers 28, among the plurality of transducers 28 of the ultrasound probe 16, on which the energy of the reflected wave is concentrated, is identified.

Similar to the positioning process, in the evaluation process also, the HIFU driver 24 causes each of the therapeutic ultrasound transducers 14 to transmit an evaluation ultrasound which is an ultrasound having lower intensity than that during the therapy. While the evaluation ultrasound converges at the focal point F, a part of the evaluation ultrasound is reflected within the body of the patient, and propagates in a direction toward the transducers 28.

Referring again to FIGs. 1 and 2, the transducers 28-1 to 28-n receive ultrasounds reflected within the body of the patient, generate respective electric signals RS1 to RSn according to the received ultrasounds, and output the electric signals to the ultrasound transmission and reception unit 20. The ultrasound transmission and reception unit 20 measures magnitudes of the electric signals RS1 to RSn, and outputs measurement values U1 to Un to the controller 22.

The controller 22 fits a distribution function to n measurement points on a scatter diagram on which values corresponding to the positions x = x1 to xn on the horizontal axis are measurement values U1 to Un, respectively. The distribution function may be, for example, a function in which an offset B is added to the Gaussian function. More specifically, the controller 22 determines a coefficient P of the Gaussian function, a center value $\mu$, a standard deviation $\sigma$, and the offset B as approximation parameters such that the curve of the function is approximated to the n measurement points on the scatter diagram through nonlinear least squares regression such as Levenberg Marquardt method.

The distribution function indicating the intensity distribution of the ultrasound (arriving ultrasound) arriving at the transmission and reception surface 38 of the ultrasound probe 16 is represented as following Equation 1.

(Equation 1)

$$I(x) = P \cdot exp\left[-\frac{(x-\mu)^2}{2\sigma^2}\right] + B$$

The controller 22 determines a degree of concentration C of the arriving ultrasound according to following Equations 2 to 5. The degree of concentration C is a product of fa, fb, and fe determined respectively by Equations 3, 5, and 6.

(Equation 2)

$$C = fa \times fb \times fe$$

(Equation 3)

$$fa = 1 - exp\left(-\frac{2P}{m}\right)$$

(Equation 4)

$$m = \frac{\sum_{i=1}^{i=n} I(xi)}{n}$$

(Equation 5)

$$fb = 1 - \left(\frac{B}{m}\right)^2$$

(Equation 6)

$$fe = exp\left(-\frac{2\sigma}{P}\right)$$

Equation 4 shows an average m of n values obtained by substituting the x coordinates of the n measurement points on the scatter diagram into Equation 1. Equation 3 shows that, as the coefficient P of the Gaussian function becomes larger than the average value m, a concentration-degree factor fa reaches 1, meaning that the arriving ultrasound is concentrated at a particular position. Equation 5 shows that, as the offset B becomes smaller than the average value m, a concentration-degree factor fb reaches 1, meaning that the arriving ultrasound is concentrated at the particular position. Equation 6 shows that, as the standard deviation σ becomes smaller, a concentration-degree factor fe reaches 1, meaning that the arriving ultrasound is concentrated at the particular position. Here, the "particular position" is a position near a position at which x = μ.

The degree of concentration C reaches 1 as the coefficient P of the Gaussian function becomes larger than the average value m, as the offset B becomes smaller than the average value m, and as the standard deviation σ becomes smaller, meaning that the arriving ultrasound is concentrated at the particular position.

Alternatively, the degree of concentration C may be defined by only one of the concentration-degree factors fa, fb, or fe. Alternatively, the degree of concentration C may be defined by a product of two of the concentration-degree factors fa, fb, or fe.

The controller 22 causes the display device 26 to display alert information when the degree of concentration C exceeds a judgment value D. The alert information indicates that there is a possibility that the reflected waves are concentrated as the arriving ultrasounds on particular transducers 28 of the ultrasound probe 16. The user referring to the alert information may avoid the concentration of the reflected waves at the particular positions by adjusting the positional relationship between the patient and the HIFU transducer unit 10, or through other means.

Alternatively, the controller 22 may cause the display device 26 to display at least one of the n measurement points in the scatter diagram, the intensity distribution, the approximation parameters, or the degree of concentration C.

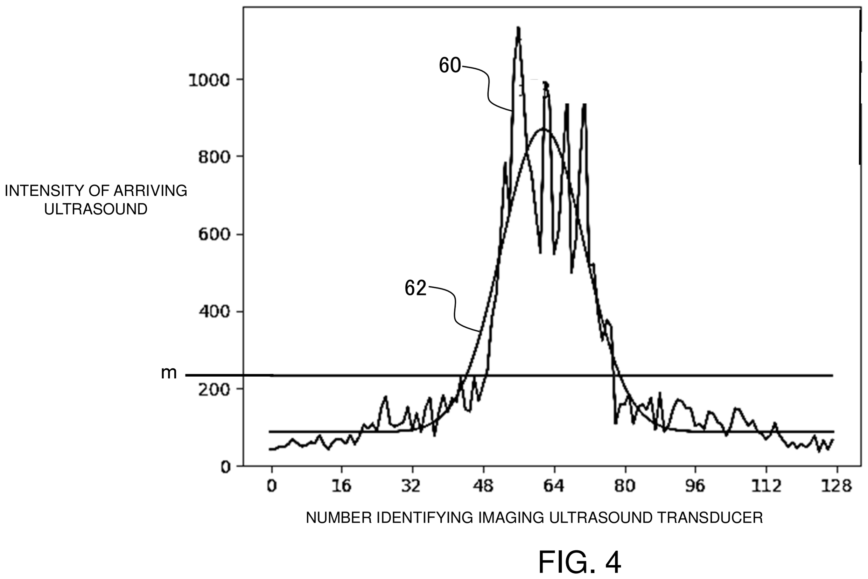

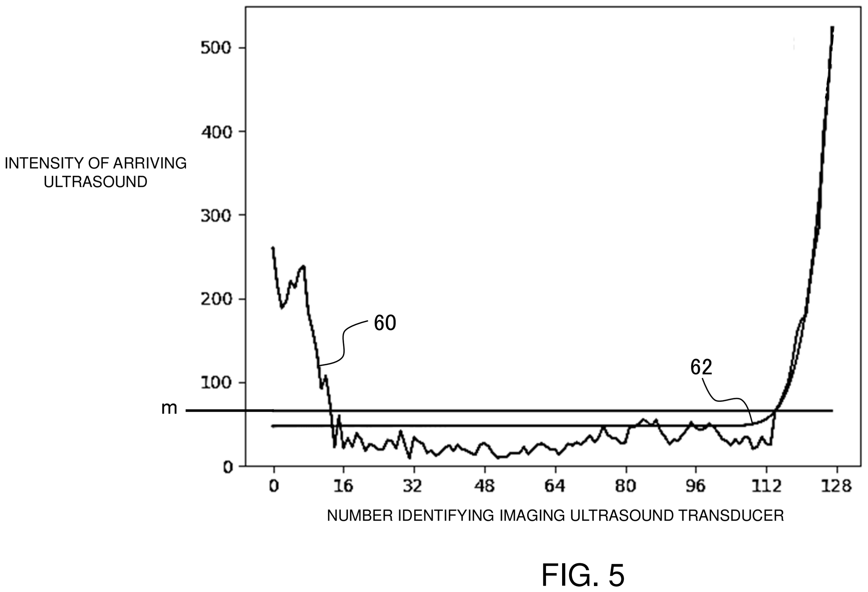

FIGs. 4 and 5 show examples in which a distribution function I(x) represented by Equation 1 is fitted to a plurality of measurement points acquired for a plurality of transducers 28 of the ultrasound probe 16. An intensity distribution 60 in each of FIGs. 4 and 5 is a distribution in which a plurality of measurements points in which the measurement values are correlated to x-coordinate values of the plurality of transducers 28 are connected by curves. An intensity distribution 62 is a distribution obtained by fitting the distribution function I(x) to the intensity distribution 60.

In an example configuration shown in FIG. 4, through the fitting, approximation parameters are determined with the coefficient P = 784.526, the center value $\mu$ = 62.090, the standard deviation $\sigma$ = 9.521, and the offset B = 87.189. In addition, the average value m = 232.322 is calculated. With these parameters, the concentration-degree factors are fa = 0.996, fb = 0.926, and fe = 0.905, and the degree of concentration is calculated as C = 0.834. In the example configuration shown in FIG. 4, the arriving ultrasounds are concentrated on the transducers 28 labeled with numbers 51 to 70. Therefore, by setting an appropriate judgment value D, alert information may be displayed on the display device 26 for the example configuration shown in FIG. 4.

In an example configuration shown in FIG. 5, through the fitting, approximation parameters are determined with the coefficient P = 1227.765, the center value $\mu$ = 139.820, the standard deviation $\sigma$ = 8.555, and the offset B = 47.3. In addition, the average value m = 66.306 is calculated. With these parameters, the concentration-degree factors are fa = 1.000, fb = 0.490, and fe = 0.015, and the degree of concentration is calculated as C = 0.007. In the example configuration shown in FIG. 5, the ultrasound intensity is high at both ends of the probe because the transducers 28 are in contact with a body surface part of the patient via the coupling bag 18, and the ultrasound is transferred only to the transducers 28 at both ends, having a slight gap with the body surface. Typically, this state is judged as a state in which the arriving ultrasounds are not concentrated. Therefore, by setting an appropriate judgment value D, the alert information is not displayed on the display device 26 for the example configuration shown in FIG. 5.

For example, by setting the judgment value D at a value between the degree of concentration C in the example configuration of FIG. 4 and the degree of concentration C in the example configuration of FIG. 5, alert information for preventing the thermal stress to the ultrasound imaging probe can be displayed on the display device 26.

In this manner, the controller 22 of the HIFU sonication apparatus 100 of the present embodiment executes an ultrasound transmission process, an ultrasound reception process, and a distribution measurement process. The ultrasound transmission process is a process to cause the therapeutic ultrasound transducer 14 to transmit an evaluation ultrasound having lower intensity than that of the therapeutic ultrasound. The ultrasound reception process is a process to acquire from each transducer 28 the reception signal based on the ultrasound received by the respective transducer 28. The distribution measurement process is a process to measure the intensity distribution of the ultrasounds arriving at the plurality of transducers 28, based on the reception signal acquired from each of the transducers 28.

The controller 22 executes processes to determine the degree of concentration C of the ultrasounds received by the transducers 28, based on the intensity distribution in regard to a direction of arrangement of the plurality of transducers 28, and to evaluate the influence of the therapeutic ultrasound on the transducers 28 based on the degree of concentration C.

The plurality of transducers 28 may be arranged in a straight line. The intensity distribution may be represented by a distribution function having one peak for a value on a vertical axis with respect to a horizontal axis which extends in the direction of arrangement of the plurality of transducers 28. Alternatively, the controller 22 may execute the evaluation process to evaluate the influence of the therapeutic ultrasound on the transducers 28 by the approximation parameters which define the distribution function. The evaluation process is a process to evaluate the influence of the therapeutic ultrasound on the transducers 28, based on the intensity distribution. The influence of the therapeutic ultrasound on the transducers 28 includes influences such as that the therapeutic ultrasound is concentrated on a particular transducer 28, and operation characteristics of the particular transducer 28 change as a result thereof.

The controller 22 evaluates the state of the HIFU sonication apparatus 100 through a method including the following steps (i) to (iv). Each step may be executed, according to a manipulation of the user, by a computing device such as a computer provided external to the HIFU sonication apparatus 100 and connected to the controller 22.

The method includes:

(i) a step in which a therapeutic ultrasound transducer 14 is caused to transmit an evaluation ultrasound having lower intensity than that of a therapeutic ultrasound;

(ii) a step in which reception signals based on ultrasounds received by transducers 28 are acquired from the transducers 28;

(iii) a step in which an intensity distribution of ultrasounds arriving at the plurality of transducers 28 is measured based on the receptions signals acquired from the transducers 28; and

(iv) a step in which a degree of concentration of ultrasounds received by the transducers 28 is determined based on the intensity distribution in regard to a direction of arrangement of the plurality of transducers 28.

Alternatively, for the distribution function I(x), in place of the Gaussian function, a function may be employed in which an offset B is added to the Lorentz function as shown by Equation 7. The Lorentz function shown in Equation 7 shows a distribution centered at the position of x = μ, and reaching the offset B according to an inverse of a quadric function as the x-coordinate value moves away from the center value μ. A kurtosis k in Equation 7 is a positive number showing a degree of how the distribution becomes convex upward as the value becomes smaller. The kurtosis k may be a value calculated based on a normal distribution.

(Equation 7)

$$I(x) = \frac{P}{k^2 + (x - \mu)^2} + B$$

When the Lorentz function shown in Equation 7 is used as the distribution function, the degree of concentration C may be one of the concentration-degree factors fa and fb, or a product of the concentration-degree factors fa and fb.

[Configuration of Present Disclosure]

Configuration 1:

A HIFU sonication apparatus comprising:

a therapeutic ultrasound transducer that transmits a therapeutic ultrasound;
an ultrasound probe having a plurality of transducers; and
a controller configured to control the therapeutic ultrasound transducer and the ultrasound probe, wherein
the controller is configured to execute:

an ultrasound transmission process to cause the therapeutic ultrasound transducer to transmit an evaluation ultrasound having lower intensity than that of the therapeutic ultrasound;
an ultrasound reception process to acquire from each of the transducers a reception signal based on an ultrasound received by each of the transducers; and
a distribution measurement process to measure an intensity distribution of ultrasounds arriving at the plurality of transducers, based on the reception signal acquired from each of the transducers.

Configuration 2:

The HIFU sonication apparatus according to configuration 1, wherein
the controller is configured to execute a process to determine a degree of concentration of the ultrasound received by each of the transducers based on the intensity distribution in regard to a direction of arrangement of the plurality of transducers.

Configuration 3:

The HIFU sonication apparatus according to configuration 1 or 2, wherein

the intensity distribution is represented by a distribution function having one peak for a value on a vertical axis with respect to a horizontal axis which extends in a direction of arrangement of the plurality of transducers, and
the controller is configured to execute an evaluation process to evaluate an influence of the therapeutic ultrasound on each of the transducers by approximation parameters which define the distribution function.

Configuration 4:

The HIFU sonication apparatus according to configuration 3, wherein
the distribution function includes a Gaussian function or a Lorentz function.

Configuration 5:

A method of evaluating a state of a HIFU sonication apparatus comprising a therapeutic ultrasound transducer which transmits a therapeutic ultrasound, and an ultrasound probe having a plurality of transducers, the method comprising:

causing the therapeutic ultrasound transducer to transmit an evaluation ultrasound having lower intensity than that of the therapeutic ultrasound;
acquiring from each of the transducers a reception signal based on an ultrasound received by each of the transducers; and
measuring an intensity distribution of ultrasounds arriving at the plurality of transducers, based on the reception signal acquired from each of the transducers.

Configuration 6:

The method according to configuration 5, further comprising:
determining a degree of concentration of the ultrasound received by each of the transducers, based on the intensity distribution in regard to a direction of arrangement of the plurality of transducers.

REFERENCE SIGNS LIST

10 HIFU transducer unit, 12 housing, 14 therapeutic ultrasound transducer, 16 ultrasound imaging probe (ultrasound probe), 18 coupling bag, 20 ultrasound transmission and reception unit, 22 controller, 24 HIFU driver, 26 display device, 28, 28-1 to 28-n imaging ultrasound transducer (transducer), 38 transmission and reception surface, 40, 42 straight line, 44 reflecting tissue, 50 B mode image, 60, 62 intensity distribution.

**Claims**

1. A HIFU sonication apparatus comprising:

a therapeutic ultrasound transducer that transmits a therapeutic ultrasound;
an ultrasound probe having a plurality of transducers; and
a controller configured to control the therapeutic ultrasound transducer and the ultrasound probe, wherein
the controller is configured to execute:

an ultrasound transmission process to cause the therapeutic ultrasound transducer to transmit an evaluation ultrasound having lower intensity than that of the therapeutic ultrasound;
an ultrasound reception process to acquire from each of the transducers a reception signal based on an ultrasound received by each of the transducers; and
a distribution measurement process to measure an intensity distribution of ultrasounds arriving at the plurality of transducers, based on the reception signal acquired from each of the transducers, and
the controller is configured to execute a process to determine a degree of concentration of the ultrasound received by each of the transducers, based on the intensity distribution in regard to a direction of arrangement of the plurality of transducers.

2. The HIFU sonication apparatus according to claim 1, wherein

the intensity distribution is represented by a distribution function having one peak for a value on a vertical axis with respect to a horizontal axis which extends in the direction of arrangement of the plurality of transducers, and
the controller is configured to execute an evaluation process to evaluate an influence of the therapeutic ultrasound on each of the transducers by approximation parameters which define the distribution function.

3. A HIFU sonication apparatus comprising:

a therapeutic ultrasound transducer that transmits a therapeutic ultrasound;
an ultrasound probe having a plurality of transducers; and
a controller configured to control the therapeutic ultrasound transducer and the ultrasound probe, wherein the controller is configured to execute:

an ultrasound transmission process to cause the therapeutic ultrasound transducer to transmit an evaluation ultrasound having lower intensity than that of the therapeutic ultrasound;
an ultrasound reception process to acquire from each of the transducers a reception signal based on an ultrasound received by each of the transducers; and
a distribution measurement process to measure an intensity distribution of ultrasounds arriving at the plurality of transducers, based on the reception signal acquired from each of the transducers,
the intensity distribution is represented by a distribution function having one peak for a value on a vertical axis with respect to a horizontal axis which extends in a direction of arrangement of the plurality of transducers, and
the controller is configured to execute an evaluation process to evaluate an influence of the therapeutic ultrasound on each of the transducers by approximation parameters which define the distribution function.

4. The HIFU sonication apparatus according to claim 2 or 3, wherein
the distribution function includes a Gaussian function or a Lorentz function.

5. A method of evaluating a state of a HIFU sonication apparatus comprising a therapeutic ultrasound transducer which transmits a therapeutic ultrasound, and an ultrasound probe having a plurality of transducers, the method comprising:

causing the therapeutic ultrasound transducer to transmit an evaluation ultrasound having lower intensity than that of the therapeutic ultrasound;
acquiring from each of the transducers a reception signal based on an ultrasound received by each of the transducers;
measuring an intensity distribution of ultrasounds arriving at the plurality of transducers, based on the reception signal acquired from each of the transducers; and
determining a degree of concentration of the ultrasound received by each of the transducers, based on the intensity distribution in regard to a direction of arrangement of the plurality of transducers.

6. The method according to claim 5, wherein

the intensity distribution is represented by a distribution function having one peak for a value on a vertical axis with respect to a horizontal axis which extends in the direction of arrangement of the plurality of transducers, and
the method further comprises evaluating an influence of the therapeutic ultrasound on each of the transducers by approximation parameters which define the distribution function.

7. A method of evaluating a state of a HIFU sonication apparatus comprising a therapeutic ultrasound transducer which transmits a therapeutic ultrasound, and an ultrasound probe having a plurality of transducers, the method comprising:

causing the therapeutic ultrasound transducer to transmit an evaluation ultrasound having lower intensity than that of the therapeutic ultrasound;
acquiring from each of the transducers a reception signal based on an ultrasound received by each of the transducers; and
measuring an intensity distribution of ultrasounds arriving at the plurality of transducers, based on the reception signal acquired from each of the transducers, wherein
the intensity distribution is represented by a distribution function having one peak for a value on a vertical axis with respect to a horizontal axis which extends in a direction of arrangement of the plurality of transducers, and
the method further comprises evaluating an influence of the therapeutic ultrasound on each of the transducers by approximation parameters which define the distribution function.

8. The method according to claim 6 or 7, wherein
the distribution function includes a Gaussian function or a Lorentz function.

EP 4 744 598 A1

RS1～RSn

100

10
12
14
16
18
A
Q(F)
28

ULTRASOUND
TRANSMISSION
AND
RECEPTION
UNIT
20

U1～Un

HIFU DRIVER
24

CONTROLLER
22

DISPLAY
DEVICE
26

FIG. 1

FIG. 2

FIG. 3

EP 4 744 598 A1

FIG. 4

FIG. 5

EP 4 744 598 A1

## INTERNATIONAL SEARCH REPORT

| International application No. |
| --- |
| **PCT/JP2024/023518** |

**A.      CLASSIFICATION OF SUBJECT MATTER**

*A61B 8/00*(2006.01)i; *A61N 7/02*(2006.01)i
FI:    A61B8/00; A61N7/02

According to International Patent Classification (IPC) or to both national classification and IPC

**B.      FIELDS SEARCHED**

Minimum documentation searched (classification system followed by classification symbols)

A61B8/00-8/15; A61B17/00-17/94; A61N7/00-7/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.      DOCUMENTS CONSIDERED TO BE RELEVANT**

| Category* | Citation of document, with indication, where appropriate, of the relevant passages | Relevant to claim No. |
| --- | --- | --- |
| A | JP 2013-55984 A (HITACHI ALOKA MEDICAL LTD.) 28 March 2013 (2013-03-28)<br>entire text, all drawings | 1-8 |
| A | JP 2014-30509 A (HITACHI MEDICAL CORPORATION) 20 February 2014 (2014-02-20)<br>entire text, all drawings | 1-8 |
| A | WO 2023/106740 A1 (IMGT CO., LTD.) 15 June 2023 (2023-06-15)<br>entire text, all drawings | 1-8 |
| A | EP 3366350 A1 (THERACLION SA) 29 August 2018 (2018-08-29)<br>entire text, all drawings | 1-8 |

☐ Further documents are listed in the continuation of Box C.      ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **02 August 2024** | **13 August 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/023518**

| Patent document cited in search report | | | Publication date (day/month/year) | Patent family member(s) | | | Publication date (day/month/year) |
|---|---|---|---|---|---|---|---|
| JP | 2013-55984 | A | 28 March 2013 | (Family: none) | | | |
| JP | 2014-30509 | A | 20 February 2014 | (Family: none) | | | |
| WO | 2023/106740 | A1 | 15 June 2023 | US | 2024/0075322 | A1 | |
| | | | | EP | 4282471 | A1 | |
| | | | | CN | 116829230 | A | |
| | | | | KR | 10-2486574 | B1 | |
| EP | 3366350 | A1 | 29 August 2018 | (Family: none) | | | |

Form PCT/ISA/210 (patent family annex) (July 2022)

**EP 4 744 598 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H871069 A
- JP 2014030509 A